# EUROPEAN PATENT APPLICATION

(11) **EP 3 845 206 A1**
(43) Date of publication of application: **07.07.2021**
(21) Application number: 18931925.4
(22) Date of filing: 30.08.2018
(51) Int. Cl.: A61F 2/66

(54) **PROSTHETIC FOOT AND CONNECTOR FOR PROSTHETIC FOOT**

(71) Applicant: Xiborg Inc., Tokyo, 135-0061 (JP)
(72) Inventor: ENDO, Ken, Tokyo 135-0061 (JP)
(74) Representative: EIP
(86) International application number: PCT/JP2018/032230
(87) International publication number: WO 2020/044519

(57) **Abstract**

A prosthetic leg and a prosthetic leg connector are provided, which are capable of enhancing the strength of a plate spring while reducing the weight thereof, as well as adjusting an attachment position of a socket with respect to the plate spring. The prosthetic leg comprises a connector and a plate spring, wherein the plate spring includes a grounding portion coming into contact with the ground, a connecting portion connected to the connector, and a curved portion curving between the grounding portion and the connecting portion, the curved portion curves in only one direction, the grounding portion is formed at one end of the curved portion, and the connecting portion is formed at another end of the curved portion substantially linearly without curving with respect to the curved portion, and includes a connecting surface formed on an inside of a curve of the curved portion, the connecting surface being connected to the connector, and the connector includes an attachment surface that is connected to the socket, the attachment surface being formed at an acute angle with respect to the connecting surface.

## Description

### Technical Field

The present invention relates to a prosthetic leg and a prosthetic leg connector.

### Background Art

There have conventionally been known sports-specific prosthetic legs, mainly for track and field, that use plate spring members molded from fiber-reinforced resins such as carbon fiber. Such prosthetic legs are each composed of a plate spring, a socket attached to the thigh or lower leg of a user, and a connector that connects the plate spring and the socket attached to the plate spring. The plate spring includes a curved portion having a grounding portion and a straight portion above the curved portion (see Patent Document 1, for example). The straight portion is for adjusting the attachment position of the connector in accordance with the height of the user and the length of the thigh or lower leg.

### Citation List

### Patent Document

Patent Document 1: Patent Publication JP-A-2018-627

### Summary

### Technical Problem

The straight portion of the plate spring has a function of adjusting, lengthwise, the attachment position of the connector, which connects the plate spring and the socket, in accordance with the user. However, the straight portion has the following issues. That is, with the straight portion being provided, the weight of the plate spring inevitably increases. Moreover, in order for the straight portion to exert the function of lengthwise adjustment as described above, the straight portion has to be formed in the vertical direction with respect to the curved portion, and as a result, the bending direction of the curved portion differs from the bending direction from the bent portion toward the straight portion. Consequently, stress is concentrated on the bent section extending from the bent portion to the straight portion, and separation therebetween may be caused in this section.

Therefore, an object of the preset invention is to provide a prosthetic leg and a prosthetic leg connector that are capable of enhancing the strength of a plate spring while reducing the weight thereof, as well as adjusting an attachment position of a socket with respect to the plate spring.

### Solution to Problem

A prosthetic leg according to one aspect of the present invention is a prosthetic leg including a connector and a plate spring, wherein the plate spring includes a grounding portion coming into contact with the ground, a connecting portion connected to the connector, and a curved portion curving between the grounding portion and the connecting portion, the curved portion curves in only one direction, the grounding portion is formed at one end of the curved portion, and the connecting portion is formed at another end of the curved portion substantially linearly without curving with respect to the curved portion, and includes a connecting surface formed on an inside of a curve of the curved portion, the connecting surface being connected to the connector, and the connector includes an attachment surface that is connected to the socket, the attachment surface being formed at an acute angle with respect to the connecting surface.

According to this aspect, the curved portion is configured to curve in only one direction, and is not provided with a straight portion extending upward, unlike the one provided in the conventional plate spring. Therefore, the reduction of the weight of the plate spring and enhancement of the strength of the plate spring have been improved. In addition, instead of providing such straight portion, the connecting surface and the attachment surface of the connector are formed at an acute angle, making it possible to provide the attachment surface of the connector in the vertical direction (perpendicular direction). By implementing vertical positional adjustment in attachment of the socket to the attachment surface, a position for attaching even the plate spring that does not have the straight portion to the socket can easily be adjusted.

The attachment surface may be formed substantially perpendicularly. According to this aspect, the same function as that of the straight portion of the conventional plate spring can be exerted by the attachment surface of the connector.

The attachment surface may have a position at which the socket is attached, with the position being adjustable in a plurality of places. According to this aspect, since vertical positional adjustment can be implemented in attachment of the socket to the attachment surface, the lengthwise adjustment that is conventionally performed with the straight portion of the plate spring can be performed with the connector.

A connector according to another aspect of the present invention includes a connecting surface connected to the prosthetic leg, and an attachment surface connected to a socket, wherein the connecting surface and the attachment surface are arranged at an acute angle.

According to this aspect, by implementing vertical positional adjustment in attachment of the socket to the attachment surface, as described above , the position for attaching the plate spring to the socket can easily be adjusted, the plate spring not having the straight portion.

The attachment surface may have a position at which the socket is attached, with the position being adjustable in a plurality of places. According to this aspect, since vertical positional adjustment can be implemented in attachment of the socket to the attachment surface, as described above, the lengthwise adjustment that is conventionally performed with the straight portion of the plate spring can be performed with the connector.

### Advantageous Effects of Invention

The preset invention can provide a prosthetic leg and a prosthetic leg connector that are capable of enhancing the strength of a plate spring while reducing the weight thereof, as well as adjusting an attachment position of a socket with respect to the plate spring.

### Brief Description of Drawings

Fig. 1 shows a perspective view (A) and a side view (B) of a configuration of a plate spring used in a prosthetic leg according to an embodiment.
Fig. 2 is a diagram of the configuration of the prosthetic leg according to the embodiment.
Fig. 3 is a diagram showing one aspect of a connector used in the prosthetic leg according to the present embodiment.
Fig. 4 is a diagram showing another aspect of the connector used in the prosthetic leg according to the embodiment.
Fig. 5 is a diagram showing another aspect of the connector used in the prosthetic leg according to the embodiment.
Fig. 6 is a diagram showing another aspect of the connector used in the prosthetic leg according to the embodiment.
Fig. 7 is a diagram showing another aspect of the connector used in the prosthetic leg according to the embodiment.
Fig. 8 is a diagram showing another aspect of the connector used in the prosthetic leg according to the embodiment.
Fig. 9 is a diagram showing another aspect of the connector used in the prosthetic leg according to the embodiment.

### Description of Embodiments

A preferred embodiment of the present invention (referred to as "present embodiment", hereinafter) is now described with reference to the accompanying drawings. (Note that, in each figure, those having the same reference numerals have the same or similar configurations.)

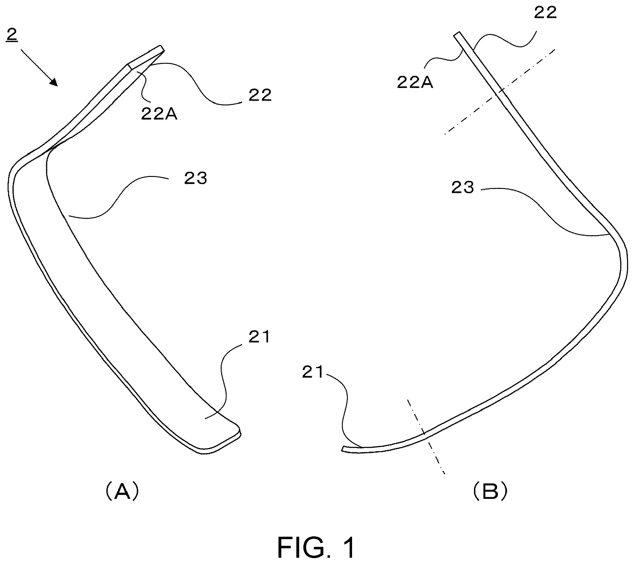

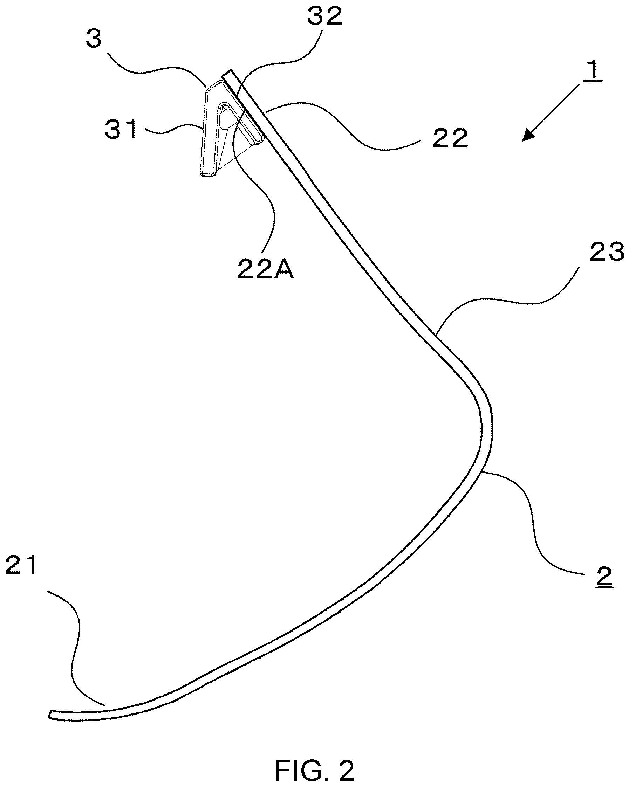

A prosthetic leg 1 of the present embodiment is obtained based on the assumption that the prosthetic leg 1 is used mainly for sports such as track and field. Fig. 1 shows a perspective view (A) and a side view (B) of a plate spring 2 used in the prosthetic leg 1 according to the present embodiment. Fig. 2 is a diagram showing the overall configuration of the prosthetic leg 1 in which a connector 3 is attached to the plate spring 2 shown in Fig. 1.

As shown in Fig. 1, the plate spring 2 is formed by molding a fiber-reinforced resin such as carbon fiber, and includes a grounding portion 21 that comes into contact with the ground, a connecting portion 22 that is connected to the connector, and a curved portion 23 that curves between the grounding portion 21 and the connecting portion 22. Note that, in Fig. 1(B), a boundary between the grounding portion 21 and the curved portion 23 and a boundary between the connecting portion 22 and the curved portion 23 are each shown by a one dot chain line.

The grounding portion 21 is formed at one end of the curved portion 23, that is, a part that comes into contact with the ground when the plate spring 2 is being used. The grounding portion 21 can be used with a spike or the like attached thereto.

The curved portion 23 is configured to curve only in one direction, that is, in a direction opposite to a direction of travel during use (leftward direction in Figs. 1 (B) and 2). In the conventional plate spring, the curved portion is configured to form an S-shaped curve in which the curved portion curves in the direction opposite to the direction of travel and then curves toward the connecting portion in the same direction as the direction of travel, to form the straight portion. On the other hand, the curved portion 23 is formed substantially linearly without curving toward the connecting portion 22.

The connecting portion 22 is formed at an end of the curved portion 23 that is opposite to the grounding portion 21, and a connecting surface 22A that comes into contact with the connector 3 is formed on the inside of a curve of the curved portion 23, that is, on the side facing the grounding portion 21. The position of the connecting surface 22A is fixed so as not to move on the connecting portion 22 of the plate spring 2.

In other words, the plate spring 2 does not have a configuration for lengthwise adjustment as in the conventional straiaht portion when connectina the connector 3. In the conventional plate spring, since the curved portion is formed into an S-shape and provided with a switching part that switches the curvature direction toward the connecting portion coming into contact with the connector, stress is concentrated on this part. However, since the plate spring 2 do not have such a part where stress is concentrated, the strength of the entire plate spring 2 high. In addition, since the plate spring 2 does not include a part corresponding to the straight portion in the first place, the weight of the entire plate spring 2 can be reduced. In a case of obtaining the same weight as that of the conventional plate spring, the plate spring 2 can be composed of a fiber-reinforced resin of higher density; high strength and high resilience can be expected.

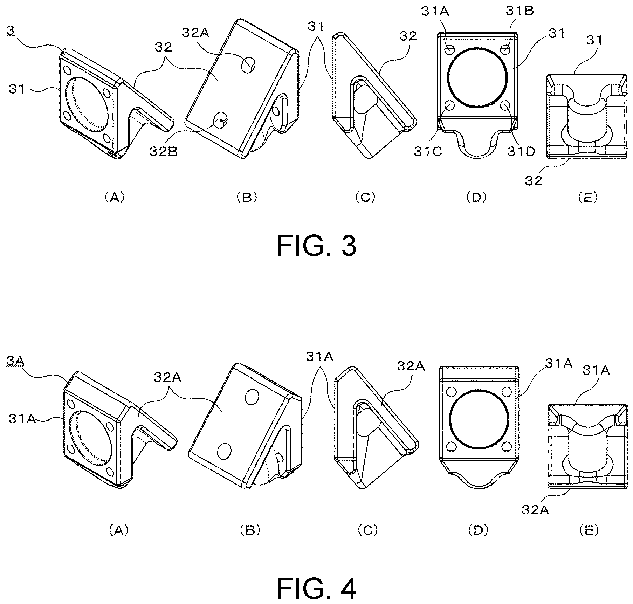

The connector 3 is produced by molding a metallic material such as titanium, aluminum, or steel. Figs. 3 to 6 are diagrams showing a detailed configuration and a plurality of aspects of the connector 3. As shown in Figs. 2 and 3, the connector 3 includes an attachment surface 31 that is connected to a socket (not shown) that is attached to the thigh or lower leg of a user, and a connecting surface 32 that is connected to the connecting portion 22 of the plate spring 2, wherein the attachment surface 31 and the connecting surface 32 are formed at an acute angle. Specifically, as shown in Fig. 2, in the plate spring 2, the curved portion 23 curves only in one direction and the connecting surface 22A of the connecting portion 22 is formed on the inside of the curve of the curved portion 23. Thus, the attachment surface 31 needs to be formed in the perpendicular direction in order to be connected to the socket. Therefore, the attachment surface 31 is configured to face in the perpendicular direction by configuring the attachment surface 31 and the connecting surface 32 of the connector 3 at an acute angle.

The connector 3 is connected to the connecting portion 22 of the plate spring 2 by means of screwing through screw holes 32A, 32B formed in the connecting surface 32, and is also connected to the socket by screwing an adapter (commercially available), not shown, through four screw holes 31A to 31D formed in the attachment surface 31, and then the socket is attached to the adapter. Note that the attachment structure itself for the connector 3, the plate spring 2, and the socket is not a significant matter in the present invention; other attachment structures can be adopted within a scope that does not deviate from the concept of the present invention.

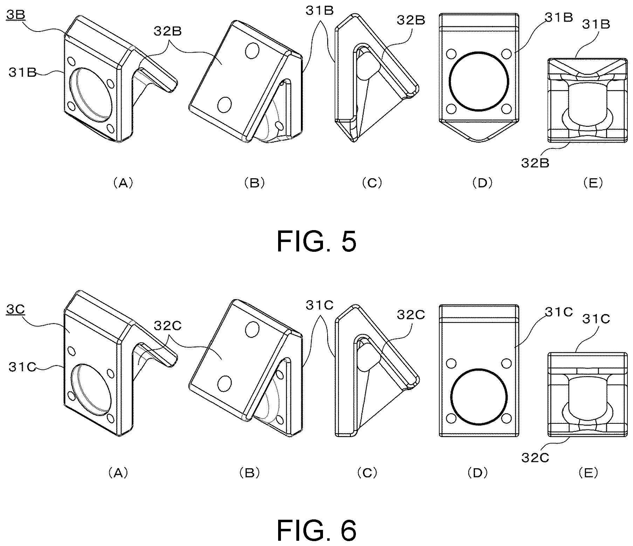

As shown as connectors 3 and 3A to 3C in Figs. 3 to 6, the connector 3 is configured in such a manner that the position of the attachment surface 31 is vertically adjustable with respect to the fixed connecting surface 32. More specifically, the attachment surface 31 shown in Fig. 3 is formed at the highest position, and the attachment surface 31 is lowered gradually from Figs. 4 to 6, wherein the attachment surface 31 shown in Fig. 6 is located at the lowest position. Thus, while the plate spring 2 has the specification in which the straight portion such as the one in the conventional plate spring is not formed and the connector 3 is firmly attached without adjusting the attachment position of the connector 3 with respect to the connecting portion 22 of the plate spring 2, the position in the connector 3 that is attached to the socket is made variable in the vertical direction, so that the adjustment according to the height of the user and the length of the thigh or lower leg can be achieved, with the plate spring 2 being commonly used.

According to the connector 3 described above, by adjusting the position of the attachment surface 31 vertically in order to connect the attachment surface 31 to the socket, the position for attaching the plate spring 2 to the socket can easily be adjusted, the plate spring 2 not having the straight portion.

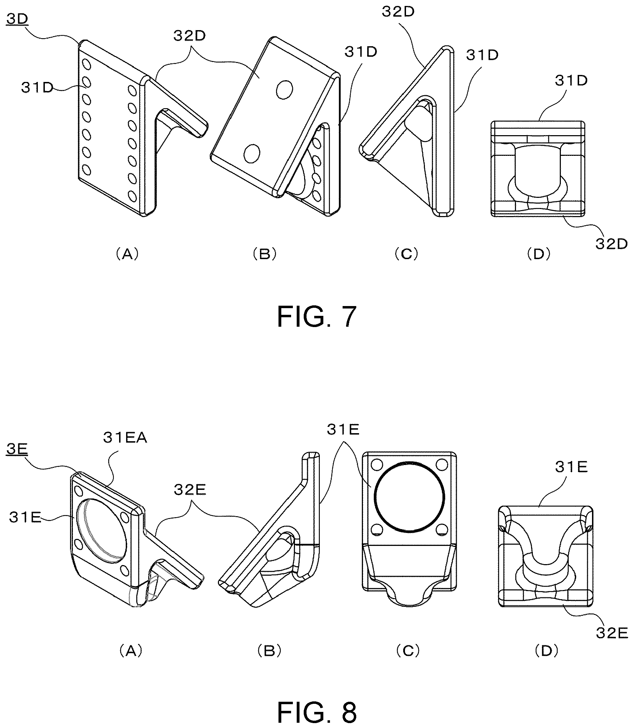

It should be noted that, as the configuration of the connector 3, for example, as shown in Fig. 7, an attachment surface 31D of a connector 3D can be made long in the vertical direction, and an attachment member for attaching the socket to the attachment surface 31D can be made vertically movable in an adaptable manner, and the position of the attachment surface in the same connector can be made vertically variable. However, since it is assumed that the prosthetic leg 1 of the present embodiment is used mainly for sports, considering that simplification and weight reduction of the configuration including the materials are the requirements, it is preferred that the prosthetic leg 1 be tailored to various sizes, as shown in Figs. 3 to 6. It is also preferred that the adjustment be made by selecting the connectors shown in Figs. 3 to 6 after confirming the position using the connector configured to vertically movable as described above.

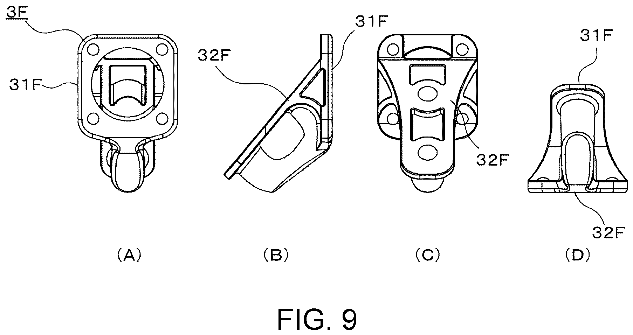

Connectors 3E and 3F shown in Figs. 8 and 9 are obtained by further improving the connectors 3 and 3A to 3C shown in Figs. 3 to 6. The connector 3E shown in Fig. 8 has a configuration in which an attachment surface 31E is made much taller than the connector 3 shown in Fig. 3, wherein when the connector 3E is attached to the plate spring 2, a top portion 31EA of the attachment surface 31E protrudes above the plate spring 2. In addition, the connector 3F shown in Fig. 8 is obtained by reducing the thickness of the attachment surface 31E of the connector 3E shown in Fig. 7 without changing the position of the attachment surface 31 E, reducing the weight of the connector 3E by scraping off parts other than the functional components while maintaining the strength.

The embodiment described above is for facilitating the understanding of the present invention and is not intended to limit the interpretation of the present invention. The components of the embodiment, as well as the arrangements, materials, conditions, shapes, size, and the like of said components are not limited to those illustrated, and therefore can be changed as appropriate. Furthermore, the configurations shown in different embodiments can be partially replaced or combined.

### Reference Signs List

- 1: Prosthetic leg
- 2: Plate spring
- 21: Grounding portion
- 22: Connecting portion
- 22A: Connecting surface
- 23: Curved portion
- 3: Connector
- 31: Attachment surface
- 32: Connecting surface

## Claims

1. A prosthetic leg comprising a connector and a plate spring,
wherein the plate spring includes a grounding portion coming into contact with the ground, a connecting portion connected to the connector, and a curved portion curving between the grounding portion and the connecting portion,
the curved portion curves in only one direction,
the grounding portion is formed at one end of the curved portion, and
the connecting portion is formed at another end of the curved portion substantially linearly without curving with respect to the curved portion, and includes a connecting surface formed on an inside of a curve of the curved portion, the connecting surface being connected to the connector, and
the connector includes an attachment surface that is connected to the socket, the attachment surface being formed at an acute angle with respect to the connecting surface.

2. The prosthetic leg according to claim 1, wherein the attachment surface is formed substantially perpendicularly.

3. The prosthetic leg according to claim 1 or 2, wherein the attachment surface has a position at which the socket is attached, with the position being adjustable in a plurality of places.

4. A connector used in a prosthetic leg, the connector comprising:
a connecting surface connected to the prosthetic leg, and an attachment surface connected to a socket, wherein
the connecting surface and the attachment surface are arranged at an acute angle.

5. The connector according to claim 4, wherein the attachment surface has a position at which the socket attached, with the position being adjustable in a plurality of places.
